# EUROPEAN PATENT SPECIFICATION

(11) **EP 4 429 533 B1**
(45) Date of publication and mention of the grant of the patent: **16.04.2025**
(21) Application number: 23790043.6
(22) Date of filing: 05.10.2023
(51) Int. Cl.: A61B 1/00, A61B 1/227

(54) **APPARATUS AND SYSTEM FOR EAR EXAMINATION**
VORRICHTUNG UND SYSTEM ZUR OHRUNTERSUCHUNG
APPAREIL ET SYSTÈME D'EXAMEN DE L'OREILLE

(30) Priority: 11.10.2022 FI 20225919
(43) Date of publication of application: 18.09.2024
(73) Proprietor: Sibbo Medical Devices Oy, 00810 Helsinki (FI)
(72) Inventor: KLOCKARS, Tuomas, 00890 Helsinki (FI)
(74) Representative: Laine IP Oy
(86) International application number: PCT/FI2023/050571
(87) International publication number: WO 2024/079386

(56) References cited:
- US-A1- 2022 233 148
- US-A1- 2022 240 771

## Description

### TECHNICAL FIELD

The present disclosure relates to an apparatus, in particular an otoscope for ear examination of a subject.

### BACKGROUND

An otoscope is used for examining the ear visually. The otoscope enables examination of various parts of the ear such as the ear canal, eardrum (the tympanic membrane) and the middle ear. Traditionally, the otoscope head contains a simple low-power magnifying lens. The lens enables the examiner to look therethrough into the ear canal. Conventionally, in most models, the lens can be removed, typically slide aside, which allows the examiner to insert instruments through the otoscope into the ear canal, such as for removing earwax or foreign bodies. However, the conventional otoscope provides a monocular vision, low magnification, and loss of magnification when the lens is moved. In addition, the length of the otoscope channel (the distance for ear cleaning) is considerably long.

A prior art document is US 2022/240771 A1 with publishing date 4 August 2022. It relates to otoscopic device for visualizing ear canal, having a two-part structure, where a handle and a knob are coupled to a control device and imaging assembly is mechanically coupled to the knob.

Therefore, in light of the foregoing discussion, there exists a need to overcome the aforementioned drawbacks associated with existing medical devices for inspection.

### SUMMARY

The present disclosure seeks to provide an apparatus for ear examination of a subject. An aim of the present disclosure is to provide a solution that overcomes at least partially the problems encountered in prior art.

The invention is defined by the independent claim 1. The dependent claims define advantageous embodiments.

In a first aspect, an embodiment of the present disclosure provides an apparatus for ear examination of a subject.

Embodiments of the present disclosure substantially eliminate or at least partially address the aforementioned problems in the prior art and provide the aforementioned apparatus for ear examination. Typically, the apparatus is designed to adapt a consumable such as an ear funnel. The apparatus is designed such that it is portable, handheld, and practical and it can be used easily. The otoscope described in the present disclosure is designed such that it is shaped and designed to be functionally better than conventional otoscopes. The shape of the novel otoscope enables better grip and stability as it can be held between the thumb and index fingers. Moreover, the apparatus is designed to allow its front chamber to be removed, hence, also referred to as "removable front chamber". The "removable front chamber" shortens access to ear canal making cleaning and other operative functions easier to perform. Moreover, the apparatus is designed to allow the at least one tool to be placed in place of the removable front chamber, the purpose of which can be to modify or personalize the otoscope/apparatus by interchanging the at least one tool.

Additional aspects, advantages, features, and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1A and 1B are the perspective views of an apparatus, in accordance with an embodiment of the present disclosure; and
FIG. 2 is a perspective view of a user holding apparatus, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, an embodiment of the present disclosure provides an apparatus for ear examination of a subject, the apparatus comprising:
- a removable front chamber; and
- a main frame having a first portion and a second portion, the first portion being adjacent to the second portion, the first portion comprising: a proximal end designed to adapt a consumable which at least partly contact an ear of the subject, and a distal end configured to receive at least partly the removable front chamber therein, and the second portion comprises a housing to accommodate electronic components, and a gripping means on an external surface of the housing, wherein the removable front chamber is configured to be removably coupled with the main frame to allow access to the ear canal.

The term "examination" as used herein refers to inspecting, analysing, or viewing ear or ear cavity of the subject with the purpose of improving it or identifying any problem thereof. Examination may be carried out by professionals such as a doctor or a nurse, or by laymen such as a caregiver or the subject himself/herself. For example, the inspection may be a visual inspection, sound emission and sound reflections that may be used for ear diagnostics, for example. It will be appreciated that the subject may be a person, such as a patient, an employee, a worker, a child, an athlete, or an animal, and the like that requires examination.

The term *"removable front chamber"* as used herein refers to a portion of the apparatus that is configured to be removably coupled with the main frame to allow access to the ear. The removable front chamber can be removed to allow shorter and better access to the ear canal. Notably, the connection between the removable front chamber and the main frame may be airtight. The term *"main frame"* as used herein refers to the main body of the apparatus that is configured to allow the removable front chamber to attach thereon. Beneficially, the main frame provides structural rigidity and better grip to the apparatus. The main frame is adapted to attach a consumable such as an ear funnel.

The term *"housing"* as used herein refers to a protective layer that is configured to surround the main frame of the apparatus. Herein, the housing contains the electronic components, and a gripping means on the external surface of the housing to hold the apparatus therefrom. Moreover, the housing provides insulation and prevents moisture and other environmental factors to affect the apparatus. The term "*gripping means*" as used herein refers to the portion on the external surface of the housing that improves the grip for holding the apparatus. Optionally, the gripping means is selected from any one of: indentation, slot, a polygonal. Advantageously, the gripping means provides a suitable location to hold the apparatus. Optionally, the gripping means may have a knurling pattern on the external surface to provide more grip. It will be appreciated that the housing is designed such that it may be used ambidextrously.

Optionally, the removable front chamber is configured to accommodate or to be replaced with at least one tool. The term *"at least one tool"* as used herein refers to a tool configured to be used for ear examination of the subject. Optionally, the removable front chamber has a cavity such that the at least one tool can be accommodated thereof. Moreover, the cavity is large enough to accommodate the at least one tool. Beneficially, the replacement of the removable front chamber to place the at least one tool increases the application of the apparatus. The cavity is configured to extend through the removable front chamber toward the distal end of the first portion when attached thereto, and to form a viewing path through the apparatus toward the subject to be examined. The cavity allows at least one of: a fixing means, a sliding lens, a light or various optical lens to be arranged thereof.

Optionally, the at least one tool comprises:
- a visual diagnostic device selected from at least one of: at least one camera, movable camera, light sources, and/or
- an acoustic diagnostic device selected from at least one of: tympanometry, acoustic reflectometry, otoacoustic emissions screener.

In this regard, the term "*visual diagnostic device*" as used herein refers to the devices that are used to produce the visual aspects of the ear. Beneficially, the visual diagnostic device provides accuracy, aids therapeutic decisions, and improves subject safety. In addition, the at least one tool comprises light sources and/or the at least one camera. Typically, the light sources are configured to provide light to illuminate the ear of the subject. Moreover, the at least one camera is configured to record or provide a live streaming of the ear of the subject while the apparatus is in use. Herein, the camera may be incorporated with a lens or without a lens. Furthermore, the at least one camera is configured to obtain an image of the ear.

The term "*acoustic diagnostic device*" as used herein refers to devices configured to produce a sound wave and sense the sound wave to identify the subject's health to determine the condition thereof. Notably, the acoustic diagnostic device includes a diagnostic audio player for music, voice or sound output and a microphone to detect sound reflections. For example, the acoustic diagnostic device emits a sound to the ear within a frequency/amplitude range such that it is able to move the tympanic membrane for example of a human or animal ear.

Optionally, the at least one tool is configured to be actuated upon receiving at least one of: a mechanical connection, an electrical connection, from the main frame. In this regard, the at least one tool is connected using the mechanical connection and the electrical connection. Typically, the mechanical connection and the electrical connection enables the functioning of the at least one tool by proving the power thereof.

Optionally, the removable front chamber is sealed to the main frame using at least one of: magnets, electromagnet, mechanical fitting with or without pneumatics or hydraulics. It will be appreciated that the apparatus is designed to provide airtightness between the removable front chamber and the frame. Notably, a means of sealing must be selected such that it is weak enough to allow removal but strong enough to provide airtightness between the removable front chamber and the frame. It will be appreciated that the means may be a magnet, a snap-fit arrangement, a slot, and the combination thereof. Notably, the means may also be used for the storage position of the apparatus when not in use. Optionally, the means may be used for power activation of the apparatus.

Optionally, any one of: the removable front chamber or the main frame comprises a pressurization system and sensor. The term "*pressurization system*" as used herein refers to the system that is configured to control the pressure within the apparatus and the exchange of air from the inside of the apparatus to the outside. Moreover, the sensor may be arranged within the removable front chamber or the frame. Typically, the sensor may be a device, module, machine, or subsystem that detects changes in pressure. The sensor may be selected from, but not limited to aneroid barometer sensor, manometer sensor, bourdon tube pressure sensor, vacuum pressure sensor, sealed pressure sensor, strain gauge sensor, variable capacitance pressure sensor, solid state pressure sensor.

Optionally, the main frame further comprises a battery, electrical components, a microphone, or a combination thereof. Notably, the battery is configured to provide power to the removable front chamber and the at least one tool. It will be appreciated that the use of the battery within the main frame makes the apparatus portable and self-sufficient. Optionally, the electrical components may be a diode, printed circuit board (PCB), transistor, resistor, inductor, capacitor, and the like. Moreover, the microphone is configured to receive the acoustic information and record thereof. Furthermore, the said record may be used the acoustic diagnostic devices.

### DETAILED DESCRIPTION OF THE DRAWINGS

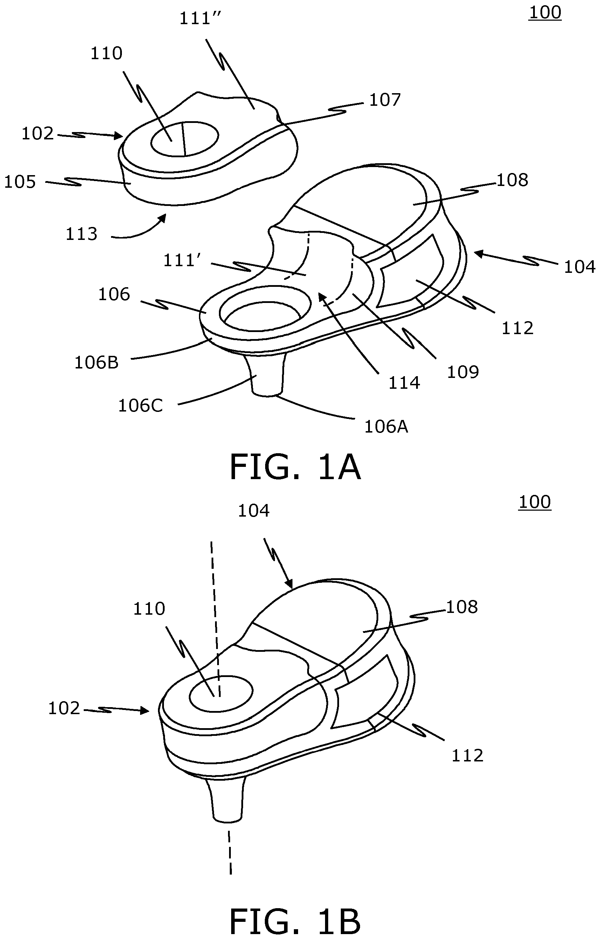

Referring to FIG. 1A and FIG. 1B, illustrated are the perspective views of an apparatus **100**, in accordance with an embodiment of the present disclosure. As shown in FIG. 1A, the apparatus **100** for ear examination comprises a removable front chamber **102** (not connected with the apparatus **100),** a main frame **104** having a first portion **106** and a second portion **108.** The first portion **106** is adjacent to and integrally formed with the second portion **108.** The first portion has a receiving surface **114** configured to receive a lower surface **113** of the removable front chamber. The receiving surface is vertically substantially lower (see FIG. 1A) than the upper surface of the second portion, which interfaces thereto via a curved side edge **109.** This allows the upper surface of the front chamber and the upper surface of the second portion of the main frame to align with each other when said parts are connected, as illustrated in FIG. 1B.

The first portion **106** further comprises a proximal end **106A** having an opening, which proximal end is designed to adapt a consumable which at least partly contact an ear of the subject, and a distal end **106B** configured to receive at least partly the removable front chamber **102** therein. A funnel-shaped part **106C** extends between the distal end and the proximal end. The proximal end, distal end and funnel-shaped part are integrally formed with the main frame and projects outwardly from the first portion, opposite the receiving surface. The second portion **108** comprises a housing to accommodate electronical components, and a gripping means **112** on an external surface of the housing. Furthermore, the removable front chamber **102** is configured to be accommodate at least one tool (not shown) or to be replaced by at least one tool. To accommodate the at least one tool, the apparatus **100** comprises a cavity **110** arranged in the removable front chamber **102.** The cavity **110** is configured to extend through the front chamber **102** toward the opposite distal end **106B** when attached thereto, and to form a viewing path through the apparatus **100** toward the subject to be examined. The cavity is designed to align with the axis of symmetry (illustrated as dashed vertical line in FIG. 1B) of the funnel-shaped part **106C,** and to provide an insertion opening for the examination instruments. The cavity **110** may further comprise, for example, a fixing means, a sliding lens, a light, or various optical lenses.

As shown in FIG. 1B, the removable front chamber **102** is sealed to the main frame **104** of the apparatus **100.** Said sealing may enable the apparatus **100** to create vacuum therein, while inspecting the subject when required. The removable front chamber **102** may be air-tightly, or optionally hermetically, sealed to the main frame **104** using at least one of: magnets, electromagnet, mechanical fitting with or without pneumatics or hydraulics. The main frame **104** further comprises a battery, electrical components and a microphone arranged within the housing. As shown, the apparatus **100** comprises the cavity **110** that allows the at least one tool to accommodate thereon and to form a view path through the apparatus.

FIGs. 1A and 1B illustrate the attachment of the front chamber **102** and the main frame **104.** In FIG. 1A, the front chamber is initially removed, i.e., the front chamber is placed separately from the main frame. When attached, the front chamber is placed so that its curved front side edge **105** is directed away from the main frame and the rear side edge **107** with the projection **111"** is towards the main frame (as shown in FIG. 1A). The front chamber is then moved towards the main frame so that the lower surface **113** of the front chamber faces the receiving surface **114** of the main frame and the recess **111'** on the side edge **109** of the main frame is aligned against the projection **111'** on the rear side edge **107** of the front chamber (see FIG. 1A). When the projection **111"** is facing the recess **111'** and the front chamber **102** is brought close enough to the main frame **104,** i.e., in the area of magnetic attraction, the projection **111"** connects to the recess **111'** and the front chamber, and the front chamber is attached to the main frame under the influence of the magnetic attraction, as illustrated in FIG. 1B. In the opposite case, when the front chamber needs to be detached from the main frame, the user pulls the front chamber with a force greater than the magnetic attraction, causing the front chamber to detach from the main frame.

It is also characteristic that when the front chamber is attached to the main frame, the upper surfaces of the front chamber and the main frame align to form a smooth inclined surface that extends over the apparatus (see FIG. 1B). Likewise, it is characteristic that the side edges surrounding the apparatus, consisting of the front side edge of the front chamber and the external surface of the housing with the gripping means, align and form a smooth curved side edge that extends along the outer circumference of the apparatus around the apparatus.

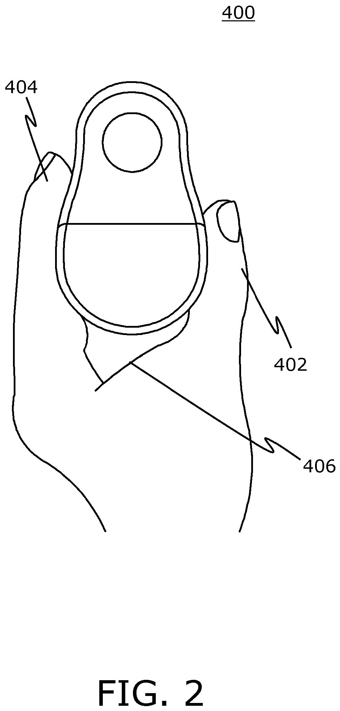

Referring to FIG. 2, illustrated is the perspective view of a user holding apparatus **400,** in accordance with an embodiment of the present disclosure. As shown, the user may hold the apparatus **400** between thumb **402** and index finger **404.** Notably, when holding the apparatus, the thumb **402** and the index finger **404** are placed on opposite sides of the apparatus **400,** on the gripping means placed on the external surface of the second portion of the frame. In the embodiment, the gripping means is shaped according to the natural shapes of the fingers. The gripping means may have a friction surface to improve grip. Without quantitatively limiting the friction, the friction of the gripping means is higher than on the housing surface between the gripping means. Furthermore, as shown, the second portion of the main frame of the apparatus **400,** is curved in shape, the curved area allows the apparatus **400** to be hold against a purlicue **406** between the thumb **402** and index finger **404,** making it feel comfortable and safe to hold the apparatus in the position intended for use. In this regard, the apparatus **400** may be used entirely by the index finger **404,** purlicue **406** and thumb **402** of the user. It is also characteristics that the upper part (in FIG. 2 the region, which is near the fingertips) is laterally narrower than the lower part (in FIG. 2 the region, which is near the purlicue, i.e., the housing). Thereby, it is achieved, among other things, that it is short vertical distance between the fingertips and the cavity of front chamber, but also short lateral distance between the index finger and the thumb, allowing the user no need for spreading the fingers, when holding the apparatus, which would require muscle tensioning.

Modifications to embodiments of the present disclosure described in the foregoing are possible without departing from the scope of the present disclosure as defined by the accompanying claims. Expressions such as "including", "comprising", "incorporating", "have", "is" used to describe, and claim the present disclosure are intended to be construed in a non-exclusive manner, namely allowing for items, components or elements not explicitly described also to be present. Reference to the singular is also to be construed to relate to the plural.

## Claims

1. An apparatus (100, 400) for ear examination of a subject, the apparatus **characterised in that** it comprises,
- a removable front chamber (102); and
- a main frame (104) having a first portion (106) and a second portion (108), the first portion (106) being adjacent to the second portion (108), the first portion (106) comprising: a proximal end (106A) designed to adapt an ear funnel which is configured to at least partly contact an ear of the subject, and a distal end (106B) configured to receive at least partly the removable front chamber (102) therein, and the second portion (108) comprising a housing to accommodate electronic components, and a gripping means (112) on an external surface of the housing, wherein the removable front chamber (108) is configured to be removably coupled with the main frame (104) to allow access to an ear canal of the subject.

2. The apparatus (100, 400) according to claim 1, wherein the removable front chamber (102) is configured to accommodate or to be replaced with at least one tool.

3. The apparatus (100, 400) according to claim 2, wherein the at least one tool comprises:
- a visual diagnostic device selected from at least one of: at least one camera, movable camera, light sources, and/or
- an acoustic diagnostic device selected from at least one of: tympanometry, acoustic reflectometry, otoacoustic emissions screener.

4. The apparatus (100, 400) according to claim 2 or 3, wherein the at least one tool is configured to be actuated upon receiving at least one of: a mechanical connection and an electrical connection, from the main frame (104).

5. The apparatus (100, 400) according to any of the preceding claims, wherein the gripping means (112) is selected from any one of: indentation, slot, a polygonal.

6. The apparatus (100, 400) according to any one of the previous claims, wherein any one of: the removable front chamber (102) or the main frame (104) comprises a pressurization system and sensor.

7. The apparatus (100, 400) according to any of the preceding claims, wherein the removable front chamber (102) is air-tightly sealed to the main frame (104) using at least one of: magnets, electromagnet, mechanical fitting with or without pneumatics or hydraulics.

8. The apparatus (100, 400) according to any of the preceding claims, wherein the main frame (104) further comprises a battery, electrical components, a microphone, or a combination thereof.

9. The apparatus (100, 400) according to any of the preceding claims, wherein the first portion (106) comprises the proximal end (106A), the distal end (106B) and a funnel-shaped part (106C) therebetween, which are integrally formed with the main frame (104) and configured to project outwardly from the first portion (106), opposite to a receiving surface (114) of the first portion (106), which is configured to receive a lower surface (113) of the removable front chamber (102).

10. The apparatus (100, 400) according to any of the preceding claims, wherein when the removable front chamber (102) is attached to the main frame (104), the upper surfaces of the removable front chamber (101) and the main frame (104) are configured to align to form a smooth inclined surface that extends over the apparatus.

11. The apparatus (100, 400) according to any of the preceding claims, wherein side edges surrounding the apparatus, consisting of a front side edge of the removable front chamber (102) and an external surface of the housing with the gripping means (112) are configured to align and form a smooth curved side edge that extends along an outer circumference of the apparatus around the apparatus.

## Patentansprüche

1. Einrichtung (100, 400) zur Ohruntersuchung eines Subjekts, wobei die Einrichtung **dadurch gekennzeichnet ist, dass** sie umfasst,
- eine abnehmbare vordere Kammer (102); und
- einen Hauptrahmen (104), der einen ersten Abschnitt (106) und einen zweiten Abschnitt (108) aufweist, wobei der erste Abschnitt (106) an den zweiten Abschnitt (108) angrenzt, wobei der erste Abschnitt (106) umfasst: ein proximales Ende (106A), das ausgelegt ist, um einen Ohrtrichter anzupassen, der konfiguriert ist, um mindestens teilweise ein Ohr des Subjekts zu berühren, und ein distales Ende (106B), das konfiguriert ist, um mindestens teilweise die abnehmbare vordere Kammer (102) darin aufzunehmen, und wobei der zweite Abschnitt (108) ein Gehäuse, um elektronische Komponenten aufzunehmen, und ein Greifmittel (112) an einer äußeren Oberfläche des Gehäuses umfasst, wobei die abnehmbare vordere Kammer (108) konfiguriert ist, um abnehmbar mit dem Hauptrahmen (104) gekoppelt zu werden, um Zugang zu einem Ohrkanal des Subjekts zu erlauben.

2. Einrichtung (100, 400) nach Anspruch 1, wobei die abnehmbare vordere Kammer (102) konfiguriert ist, um mindestens ein Werkzeug aufzunehmen oder dadurch ersetzt zu werden.

3. Einrichtung (100, 400) nach Anspruch 2, wobei das mindestens eine Werkzeug umfasst:
- eine visuelle Diagnosevorrichtung, ausgewählt aus mindestens einem von: mindestens einer Kamera, einer beweglichen Kamera, Lichtquellen und/oder
- eine akustische Diagnosevorrichtung, ausgewählt aus mindestens einem von: Tympanometrie, akustischer Reflektometrie, otoakustischem Emissions-Screener.

4. Einrichtung (100, 400) nach Anspruch 2 oder 3, wobei das mindestens eine Werkzeug konfiguriert ist, um beim Empfangen von mindestens einer von: einer mechanischen Verbindung und einer elektrischen Verbindung vom Hauptrahmen (104) betätigt zu werden.

5. Einrichtung (100, 400) nach einem der vorstehenden Ansprüche, wobei das Greifmittel (112) aus einem ausgewählt ist von: Vertiefung, Schlitz, Polygon.

6. Einrichtung (100, 400) nach einem der vorstehenden Ansprüche, wobei eine(r) von: der abnehmbaren vorderen Kammer (102) oder dem Hauptrahmen (104) ein Druckbeaufschlagungssystem und einen Sensor umfasst.

7. Einrichtung (100, 400) nach einem der vorstehenden Ansprüche, wobei die abnehmbare vordere Kammer (102) unter Verwendung von mindestens einem von: Magneten, Elektromagneten, mechanischer Montage mit oder ohne Pneumatik oder Hydraulik luftdicht am Hauptrahmen (104) befestigt ist.

8. Einrichtung (100, 400) nach einem der vorstehenden Ansprüche, wobei der Hauptrahmen (104) weiter eine Batterie, elektrische Komponenten, ein Mikrofon oder eine Kombination davon umfasst.

9. Einrichtung (100, 400) nach einem der vorstehenden Ansprüche, wobei der erste Abschnitt (106) das proximale Ende (106A), das distale Ende (106B) und einen trichterförmigen Teil (106C) dazwischen umfasst, die in einem Stück mit dem Hauptrahmen (104) gebildet sind und konfiguriert sind, um vom ersten Abschnitt (106) entgegengesetzt zu einer Aufnahmeoberfläche (114) des ersten Abschnitts (106) nach außen vorzustehen, der konfiguriert ist, um eine untere Oberfläche (113) der abnehmbaren vorderen Kammer (102) aufzunehmen.

10. Einrichtung (100, 400) nach einem der vorstehenden Ansprüche, wobei, wenn die abnehmbare vordere Kammer (102) an dem Hauptrahmen (104) angebracht ist, die oberen Oberflächen der abnehmbaren vorderen Kammer (101) und des Hauptrahmens (104) konfiguriert sind, um zu fluchten, um eine glatte, geneigte Oberfläche zu bilden, die sich über die Einrichtung erstreckt.

11. Einrichtung (100, 400) nach einem der vorstehenden Ansprüche, wobei Seitenkanten, die die Einrichtung umgeben, die aus einer vorderen Seitenkante der abnehmbaren vorderen Kammer (102) und einer Außenoberfläche des Gehäuses mit den Greifmitteln (112) bestehen, konfiguriert sind, um zu fluchten und eine glatte, gekrümmte Seitenkante zu bilden, die sich entlang eines Außenumfangs der Einrichtung um die Einrichtung herum erstreckt.

## Revendications

1. Appareil (100, 400) pour un examen de l'oreille d'un sujet, l'appareil étant **caractérisé en ce qu'**il comprend,
- une chambre avant amovible (102) ; et
- un cadre principal (104) présentant une première partie (106) et une seconde partie (108), la première partie (106) étant adjacente à la seconde partie (108), la première partie (106) comprenant : une extrémité proximale (106A) conçue pour adapter un spéculum auriculaire qui est configuré pour entrer en contact au moins partiellement avec une oreille du sujet et une extrémité distale (106B) configurée pour recevoir au moins partiellement la chambre avant amovible (102) à l'intérieur de celle-ci et la seconde partie (108) comprenant un boîtier pour loger des composants électroniques, et un moyen de préhension (112) sur une surface externe du boîtier, dans lequel la chambre avant amovible (108) est configurée pour être couplée de manière amovible au cadre principal (104) pour permettre l'accès à un conduit auditif du sujet.

2. Appareil (100, 400) selon la revendication 1, dans lequel la chambre avant amovible (102) est configurée pour accueillir ou être remplacée par au moins un outil.

3. Appareil (100, 400) selon la revendication 2, dans lequel le au moins un outil comprend :
- un dispositif de diagnostic visuel sélectionné parmi au moins un élément parmi : au moins une caméra, une caméra mobile, des sources de lumière, et/ou
- un dispositif de diagnostic acoustique sélectionné parmi au moins un élément parmi : un détecteur de tympanométrie, réflectométrie acoustique, d'émissions otoacoustiques.

4. Appareil (100, 400) selon la revendication 2 ou 3, dans lequel le au moins un outil est configuré pour être actionné lors de la réception d'au moins l'une parmi : une connexion mécanique et une connexion électrique à partir du cadre principal (104).

5. Appareil (100, 400) selon l'une quelconque des revendications précédentes, dans lequel le moyen de préhension (112) est sélectionné parmi l'un quelconque parmi : une indentation, une fente, un polygone.

6. Appareil (100, 400) selon l'une quelconque des revendications précédentes, dans lequel l'un quelconque parmi : la chambre avant amovible (102) ou le cadre principal (104) comprend un système de pressurisation et un capteur.

7. Appareil (100, 400) selon l'une quelconque des revendications précédentes, dans lequel la chambre avant amovible (102) est scellée de manière étanche à l'air au cadre principal (104) à l'aide d'au moins un élément parmi : des aimants, un électroaimant, un raccord mécanique avec ou sans pneumatique ou hydraulique.

8. Appareil (100, 400) selon l'une quelconque des revendications précédentes, dans lequel le cadre principal (104) comprend en outre une batterie, des composants électriques, un microphone ou une combinaison de ceux-ci.

9. Appareil (100, 400) selon l'une quelconque des revendications précédentes, dans lequel la première partie (106) comprend l'extrémité proximale (106A), l'extrémité distale (106B) et une partie en entonnoir (106C) entre celles-ci qui sont formées d'un seul tenant avec le cadre principal (104) et configurées pour faire saillie vers l'extérieur à partir de la première partie (106), à l'opposé d'une surface de réception (114) de la première partie (106), qui est configurée pour recevoir une surface inférieure (113) de la chambre avant amovible (102).

10. Appareil (100, 400) selon l'une quelconque des revendications précédentes, dans lequel lorsque la chambre avant amovible (102) est fixée au cadre principal (104), les surfaces supérieures de la chambre avant amovible (101) et du cadre principal (104) sont configurées pour s'aligner afin de former une surface inclinée lisse qui s'étend sur l'appareil.

11. Appareil (100, 400) selon l'une quelconque des revendications précédentes, dans lequel des bords latéraux entourant l'appareil, consistant en un bord latéral avant de la chambre avant amovible (102) et une surface externe du boîtier avec le moyen de préhension (112), sont configurés pour s'aligner et former un bord latéral incurvé lisse qui s'étend sur la circonférence extérieure de l'appareil, autour de l'appareil.
